# FASCICULE DE BREVET EUROPEEN

(11) **EP 3 148 479 B1**
(45) Date de publication et mention de la délivrance du brevet: **16.09.2020**
(21) Numéro de dépôt: 15732809.7
(22) Date de dépôt: 27.05.2015
(51) Int. Cl.: A61D 19/02

(54) **PAILLETTE POUR LA CONSERVATION D'UNE DOSE PRÉDÉTERMINÉE DE SUBSTANCE À BASE LIQUIDE, NOTAMMENT DE LA SEMENCE ANIMALE DILUÉE; ET SYSTÈME COMPORTANT UNE TELLE PAILLETTE ET UN MILIEU DE DILUTION POUR DONNER UNE TELLE SUBSTANCE**
PIPETTE ZUR BEIBEHALTUNG EINER VORBESTIMMTEN DOSIS EINER FLÜSSIGEN SUBSTANZ, INSBESONDERE VERDÜNNTES TIERSPERMA, UND SYSTEM MIT SOLCH EINER PIPETTE UND EINEM VERDÜNNUNGSMEDIUM ZUR HERSTELLUNG SOLCH EINER SUBSTANZ
STRAW FOR PRESERVING A PREDEFINED DOSE OF A LIQUID SUBSTANCE, IN PARTICULAR DILUTED ANIMAL SEMEN; AND SYSTEM COMPRISING SUCH A STRAW AND A DILUTION MEDIUM FOR PRODUCING SUCH A SUBSTANCE

(30) Priorité: 28.05.2014 FR 1454894
(43) Date de publication de la demande: 05.04.2017
(73) Titulaire: IMV Technologies, 61300 Saint-Ouen-Sur-Iton (FR)
(72) Inventeur: SCHMITT, Eric, 53700 Villaines-la-Juhel (FR)
(74) Mandataire: Santarelli
(86) Numéro de dépôt international: PCT/FR2015/051395
(87) Numéro de publication internationale: WO 2015/181495

(56) Documents cités:
- EP-A1- 2 526 768
- EP-A2- 0 922 451
- WO-A1-2013/076232
- FR-A1- 2 810 535
- US-A1- 2009 208 566

## Description

L'invention a trait d'une manière générale à la conservation d'une dose prédéterminée de substance à base liquide contenant de la matière biologique. Une telle substance est par exemple de la semence animale diluée.

L'invention concerne plus particulièrement les paillettes pour effectuer une telle conservation.

On sait que ces paillettes sont formées classiquement par un tube mince, ayant par exemple un diamètre interne de 1,6 ou 2,5 mm, et par un bouchon engagé dans le tube mince.

A l'état rempli, le bouchon est disposé au voisinage d'une première extrémité du tube et la dose de substance à base liquide est disposée dans la paillette entre le bouchon et la seconde extrémité du tube.

Pour remplir la paillette, la première extrémité du tube, voisine du bouchon, est mise en communication avec une source de vide tandis que la seconde extrémité est mise en communication avec un récipient contenant la substance à introduire dans la paillette. L'air initialement contenu entre le bouchon et la seconde extrémité est aspiré au travers du bouchon tandis que la substance progresse dans le tube jusqu'à ce qu'elle rencontre le bouchon, qu'elle ne peut franchir car il devient étanche aux liquides.

Le cas échéant, après remplissage, la paillette est soudée au voisinage de l'une ou de ses deux extrémités et est stockée au froid.

Pour vider la paillette, le cas échéant après découpage des portions d'extrémité soudées et décongélation, on fait pénétrer dans le tube par l'extrémité la plus proche du bouchon une tige qui vient porter contre le bouchon. Avec cette tige, on fait coulisser le bouchon à la façon d'un piston vers l'extrémité la plus éloignée du bouchon, de sorte que la dose de substance initialement contenue dans la paillette en est expulsée par cette extrémité.

En général, les bouchons de paillette sont du type tripartite décrit à l'origine dans le brevet français 995.878, correspondant au brevet britannique 669,265, c'est-à-dire formés par deux tampons en substance fibreuse enserrant une poudre se transformant au contact d'un liquide en une pâte ou gel imperméable adhérent à la paroi du tube pour que le bouchon soit étanche aux liquides.

La demande de brevet européen EP 0 873 726 propose que le bouchon soit fait d'un cylindre monobloc de matière microporeuse hydrophobe.

Les demandes de brevet français 2 771 285 et 2 784 572, auxquelles correspondent la demande de brevet américain US 2001/0014376 et le brevet américain US 6,416,611, proposent que le bouchon soit constitué d'un insert rigide percé d'un orifice sensiblement coaxial et d'une membrane microporeuse et hydrophobe associée à l'insert pour obturer l'orifice de l'insert du côté interne.

Les demandes de brevet français 2 824 255 et 2 824 256, auxquelles correspondent les demandes de brevet américains US 2002/0183653 et US 2002/0188222, proposent d'ajouter dans le bouchon, en outre de la poudre et des fibres, des éléments non absorbants, en l'occurrence un noyau en matière thermoplastique, revêtu d'une gaine en fils tressés, et de la matière non absorbante sous forme dispersée, dans la poudre.

Le document FR 2 810 535 divulgue un procédé de remplissage d'une paillette de stockage avec de la semence comportant un bouchon placé entre deux tampons. Dans cet état de la technique, il est fait référence à l'utilisation d'un dilueur, c'est à dire un milieu liquide non spermicide et non adhérent, assurant la conservation et/ou la nutrition de la semence. Des exemples de tels dilueurs sont à base de citrate, de jaune d'oeuf et de glycérol. Le cas échéant, il peut également contenir des ions calcium aptes à gélifier plus rapidement la poudre du bouchon quand il s'agit d'alginate. L'invention vise à limiter la perte, dans le bouchon d'une telle paillette, du produit d'intérêt contenu dans la substance à base liquide, par exemple les spermatozoïdes si la substance est de la semence diluée.

L'invention propose à cet effet une paillette pour la conservation d'une dose prédéterminée de substance à base liquide contenant de la matière biologique, comportant un tube s'étendant entre une première extrémité et une seconde extrémité et comportant un bouchon perméable aux gaz et étanche aux liquides, lequel bouchon est disposé dans le tube au voisinage de sa première extrémité et s'étend entre une première extrémité tournée vers la première extrémité du tube et une seconde extrémité tournée vers la seconde extrémité du tube ; caractérisée en ce qu'à l'état vide initial de la paillette ledit bouchon comporte un élément imprégné par des cations multivalents au moins au voisinage d'une première extrémité tournée vers la seconde extrémité du tube.

On sait que dans un milieu aqueux, les cations multivalents ont la capacité de réticuler les alginates.

Par conséquent, si la substance à base liquide contient un alginate, au contact de la seconde extrémité du bouchon de la paillette, la substance s'épaissit et forme une matrice ou un réseau qui ne peut être franchi par le produit d'intérêt, par exemple les spermatozoïdes, qui ne peut donc pas pénétrer dans le bouchon où il serait perdu.

La paillette selon l'invention permet donc de limiter la perte du produit d'intérêt dans le bouchon de la paillette.

On notera qu'il est possible d'ajouter un alginate à une substance à base liquide dans une proportion telle que l'alginate peut réagir avec les cations multivalents comme il vient d'être indiqué, sans que cet ajout influe sur la viscosité de la substance à base liquide ou alors influe sur cette viscosité de façon minime. Par conséquent, cet ajout n'influe pas ou peu sur le produit d'intérêt contenu dans la substance, par exemple les spermatozoïdes. De même, cet ajout n'influe pas ou peu sur la coopération de la substance à base liquide avec la paillette, en dehors de la réticulation susmentionnée de l'alginate.

On observera que l'imprégnation du bouchon de la paillette au moins au voisinage de sa seconde extrémité par des cations multivalents est une opération relativement simple à effectuer. La paillette selon l'invention est ainsi simple, commode et économique à produire.

Selon des caractéristiques avantageuses :
- lesdits cations multivalents sont des cations bivalents ;
- lesdits cations bivalents comportent des cations de baryum Ba⁺⁺ ;
- lesdits cations bivalents comportent des cations de calcium Ca⁺⁺ ;
- ledit bouchon comporte deux tampons en substance fibreuse enserrant un agent de scellement formé par une poudre se transformant au contact d'un liquide en une pâte ou gel imperméable adhérent à la paroi du tube pour que le bouchon soit étanche aux liquides ;
- dans le bouchon de la paillette, le tampon situé du côté de la seconde extrémité du tube forme ledit élément imprégné, avec ladite première extrémité de l'élément imprégné qui forme ladite seconde extrémité du bouchon ;
- ledit bouchon comporte un tampon barrière perméable aux gaz et aux liquides s'étendant entre une première extrémité tournée vers la première extrémité du tube et une seconde extrémité tournée vers la seconde extrémité du tube, avec la première extrémité de l'élément imprégné et la seconde extrémité du tampon barrière qui sont disposées l'une contre l'autre ;
- le tampon barrière est hydrophobe ; et/ou
- ledit élément imprégné est un tampon entièrement imprégné de cations bivalents.

L'invention vise également un système pour la conservation d'une matière biologique, caractérisé en ce qu'il comporte:
- une paillette telle qu'exposé ci-dessus pour la conservation d'une dose prédéterminée de substance à base liquide contenant de ladite matière biologique ; et
- un milieu liquide de dilution pour donner par mélange, dans des conditions prédéterminées, ladite substance à base liquide contenant de la matière biologique ; lequel milieu contient un alginate en solution.

La substance à base liquide obtenue avec ce milieu liquide contient donc un alginate et convient pour être utilisée avec une paillette telle qu'exposé ci-dessus.

De façon avantageuse, ladite substance à base liquide est de la semence animale diluée et ledit alginate est dans une concentration comprise entre 0,1 et 6 g/l ; et de façon encore plus avantageuse dans une concentration comprise entre 2,5 et 5 g/l.

L'exposé de l'invention sera maintenant poursuivi par la description détaillée d'exemples de réalisation, donnée ci-après à titre illustratif et non limitatif, en référence aux dessins annexés, sur lesquels :
- la figure 1 est une vue schématique en coupe longitudinale d'une paillette selon l'invention, à l'état vide ;
- la figure 2 est une vue semblable à la figure 1 mais montrant la paillette à l'état rempli ;
- la figure 3 est un agrandissement de la partie de la figure 2 située autour de l'extrémité du bouchon que l'on y voit à droite ;
- la figure 4 montre de façon schématique un flacon contenant un milieu liquide de dilution, un récipient contenant de la semence animale pure et un récipient contenant de la semence animale diluée obtenue par dilution de cette semence pure avec ce milieu ; et
- la figure 5 est vue partielle d'une variante de la paillette, dans laquelle le bouchon comporte en outre un tampon barrière.

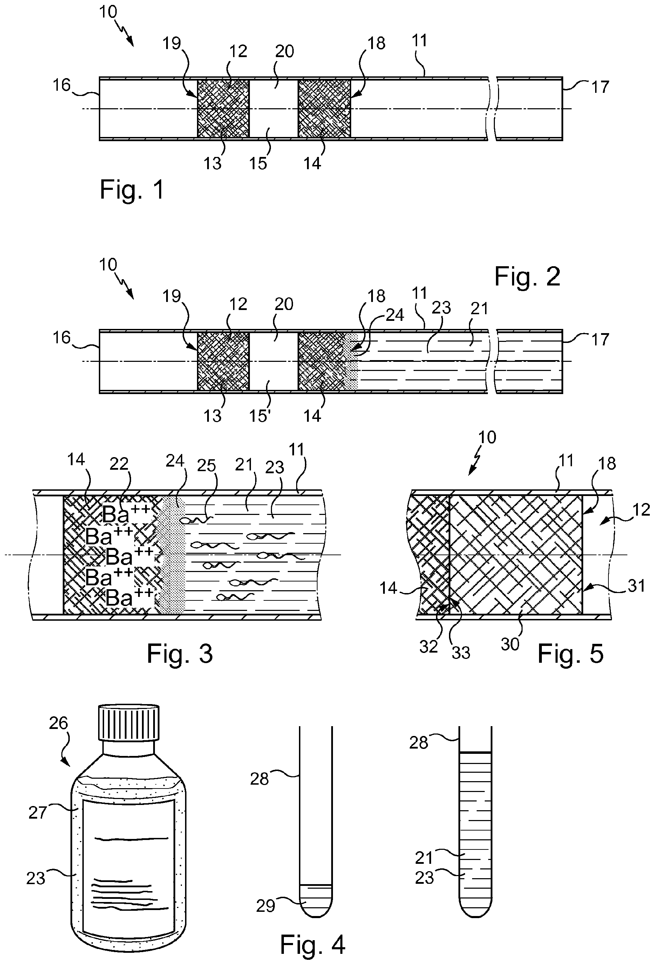

La paillette 10 illustrée sur la figure 1 comporte un tube 11 et un bouchon 12.

Le tube 11 est classiquement en matière plastique extrudée, ici transparente, avec un diamètre interne qui est par exemple de 1,6 ou de 2,5 mm et une longueur de l'ordre de 133 mm.

Le bouchon 12 est du type tripartite, c'est-à-dire formé par deux tampons 13 et 14 d'une substance fibreuse, par exemple en fils tressés, enserrant un agent de scellement 20 formé par une poudre 15 (figure 1) capable de se transformer au contact d'un liquide en une pâte ou gel 15' (figure 2) imperméable adhérant à la paroi du tube 11 pour que le bouchon 12 soit étanche aux liquides.

A l'état initial, illustré sur la figure 1, le bouchon 12 est disposé au voisinage de l'extrémité 16 du tube 11 et il est prévu qu'à l'état rempli, la dose de substance à base liquide qui doit être conservée dans la paillette 10 soit disposée entre le bouchon 12 et l'extrémité 17 du tube 11 la plus éloignée du bouchon 12.

Pour remplir la paillette 10, l'extrémité 16 est mise en communication avec une source de vide tandis que l'extrémité 17 est mise en communication avec un récipient contenant la substance à introduire dans la paillette.

L'air initialement contenu entre le bouchon 12 et l'extrémité 17 est aspiré au travers du bouchon 12 tandis que la substance 21 (figure 2) progresse dans le tube 11 jusqu'à ce qu'elle rencontre le bouchon 12, par l'extrémité 18 de celui-ci tournée vers l'extrémité 17 du tube 11, c'est-à-dire l'extrémité du bouchon 12 que l'on voit à droite sur les figures 1 et 2.

La paillette 10 est alors à l'état rempli montré sur la figure 2.

Le cas échéant, après remplissage, la paillette est soudée au voisinage de l'une ou de ses deux extrémités 16 et 17 et est stockée au froid.

Pour vider la paillette 10, le cas échéant après découpage des portions d'extrémité soudées et décongélation, on fait pénétrer dans le tube 11 une tige qui vient porter contre l'extrémité 19 du bouchon 12 (extrémité située du côté opposé à l'extrémité 18).

Avec cette tige, on fait coulisser le bouchon 12 à la façon d'un piston vers l'extrémité 17 ou l'extrémité correspondante après découpe de la portion soudée, ce qui provoque l'expulsion de la dose de substance 21 qui avait été introduite dans la paillette.

On va maintenant décrire plus en détail à l'appui de la figure 3 le tampon 14 du bouchon 12 de la paillette 10 et la zone située au voisinage de son extrémité 18 lorsque la paillette 10 est à l'état rempli montrée sur la figure 2.

Le tampon 14 est imprégné par des cations bivalents 22, ici des cations de baryum Ba⁺⁺.

La substance 21 est ici de la semence animale diluée qui contient un alginate 23.

On sait que dans un milieu aqueux, les cations bivalents tels que les cations 22, ont la capacité de réticuler les alginates tels que l'alginate 23.

Ainsi, lorsque la substance 21 est venue au contact de l'extrémité 18 du bouchon 12, la substance 21 s'est épaissie et il s'est formé une matrice ou réseau 24.

La matrice ou réseau 24 est telle que les spermatozoïdes 25 contenus dans la substance 21 ne peuvent franchir la matrice ou réseau 24.

Par conséquent, les spermatozoïdes 25 ne peuvent pas pénétrer dans le bouchon 12, où ils seraient perdus.

Ici, le tampon 14 est tout entier imprégné de cations bivalents 22, pour des raisons de commodité de fabrication.

Par exemple, le tampon 14 est fabriqué à partir d'un tronçon d'une mèche tressée qui a été trempée dans une solution saturée de sel de baryum puis retirée du bain et mise à sécher avant d'être découpée en tronçons dont l'un forme le tampon 14.

En variante, seule la région à proximité de l'extrémité 18 est imprégnée de cations bivalents 22.

La figure 4 montre un flacon 26 contenant un milieu de dilution 27, un récipient 28 contenant de la semence animale pure 29 et le même récipient 28 contenant de la semence animale diluée, formant la substance 21, obtenue par dilution de la semence animale pure 29 avec le milieu de dilution 27.

Il est bien entendu que le milieu de dilution 27 n'a pas encore été mélangé avec de la semence pure, et est donc distinct de la semence diluée.

Le milieu de dilution 27 est par exemple celui commercialisé par la demanderesse sous le nom de OptiXcell.

La dilution de la semence pure 29 par le milieu 27 se fait dans des conditions prédéterminées, notamment en ce qui concerne le rapport de dilution, c'est-à-dire le rapport entre le volume total de la semence diluée 21 et le volume de la semence pure 29.

Le milieu de dilution 27 contient l'alginate 23, qui a été sélectionné pour réticuler au contact des cations bivalents 22 afin de former la matrice ou réseau 24, qui est donc une zone épaissie de la semence diluée 21.

Une plage de concentration qui convient particulièrement bien est la plage allant de 0,1 g à 6 g d'alginate par litre de milieu de dilution 27. Une plage particulièrement avantageuse s'étend entre 2,5 g/l et 5 g/l.

Par exemple, avec une concentration en alginate de l'ordre de 2,5 g/l la proportion de substance à base liquide perdue dans le bouchon 12 est de 3 à 4 % ; et avec une concentration de l'ordre de 5 g/l, la proportion de substance à base liquide perdue dans le bouchon tel que 12 est de l'ordre de 1 à 3 %.

On notera que cette proportion de liquide perdue dans le bouchon est déterminée en faisant la différence entre la quantité de substance à base liquide introduite dans la paillette jusqu'à ce que le bouchon 12 devienne étanche aux liquides et la quantité de liquide que peut restituer la paillette en faisant coulisser le bouchon 12 comme expliqué ci-dessus.

On notera que sur les figures 2 et 3, la matrice ou réseau 24 est montrée uniquement à proximité de l'extrémité 18 du bouchon 12.

Suivant la concentration du milieu 27 en alginate, au lieu d'avoir une présence localisé de la matrice ou réseau 24, ainsi qu'illustré sur les figures 2 et 3, la matrice ou réseau peut s'étendre jusqu'à une distance relativement importante de l'extrémité 18 du bouchon 12.

La variante de la paillette 10 montrée sur la figure 5 est semblable à la paillette 10 montrée sur les figures 1 à 3 si ce n'est que le bouchon 12 comporte en outre un tampon barrière 30 du côté de l'extrémité 17 du tube 11.

Le tampon barrière 30 s'étend entre une extrémité 31 tournée vers l'extrémité 17 du tube 11 et une extrémité 32 tournée vers l'extrémité 16 du tube 11.

Dans la paillette 10 montrée sur les figures 1 à 3 l'extrémité 18 du bouchon 12 tournée vers l'extrémité 17 du tube 11 fait partie du tampon 14. Dans la variante de la paillette 10 montrée sur la figure 5, l'extrémité 18 fait partie du tampon barrière 30 et le tampon 14 a, du côté tourné vers l'extrémité 17, une extrémité 33 distincte de l'extrémité 18.

L'extrémité 33 du tampon 14 et l'extrémité 32 du tampon barrière 30 sont disposées l'une contre l'autre.

Le tampon barrière 30 est fibreux. Il est perméable aux gaz et aux liquides.

La paillette 10 montrée sur la figure 5 s'utilise de la même façon que la paillette 10 montrée sur les figures 1 à 3.

Lors du remplissage, la substance 21 traverse le tampon barrière 30 et rencontre le tampon 14 par son extrémité 33 tournée vers l'extrémité 17 du tube 11. De même que pour la paillette 10 montrée sur les figures 1 à 3, la substance 21 s'épaissit et il se forme la matrice ou réseau 24.

Le tampon barrière 30 permet à la matrice ou réseau 24 de rester dans le bouchon 12 : le tampon barrière 30 empêche le passage de la matrice ou réseau 24 vers la substance 21.

Le tampon barrière 30 est ici une tresse formée par des fils agencés en une âme et une couverture entourant l'âme.

Chaque fil est hydrophobe. Par conséquent, le tampon barrière 30 est hydrophobe, et a donc un effet répulsif sur l'eau.

Cet effet répulsif n'empêche pas la substance 21 de traverser le tampon barrière 30 et d'atteindre le tampon imprégné 14, puisqu'en pratique la substance 21 vient à la rencontre du bouchon 12 avec une certaine vitesse.

Au cours du passage de la substance 21 dans le tampon barrière 30, les fils qui le forment n'absorbent pas de liquide ; et après que le passage de la substance 21 est bloqué par le bouchon 12, le tampon barrière 30 ne garde pas le liquide situé dans ses interstices mais le refoule dans la dose de substance liquide située entre l'extrémité 31 du tampon barrière 30 et l'extrémité 17 du tube 11.

Par conséquent, il n'y a pas de consommation ou alors une consommation très réduite de substance liquide par le tampon barrière 30.

Dans une variante non illustrée, en particulier lorsque la concentration en alginate dans le milieu de dilution 27 est relativement élevée, le bouchon 12 tripartite est remplacé par un bouchon formé uniquement par un tampon tel que le tampon 14.

Dans une variante non illustrée, le bouchon 12 est agencé différemment, par exemple fait d'un cylindre monobloc de matière microporeuse hydrophobe telle que décrit dans la demande de brevet européen EP 0 873 726.

Dans des variantes non illustrées, les cations bivalents 22 formés par des ions baryum Ba⁺⁺ sont remplacés par d'autres cations bivalents, par exemple des cations calcium Ca⁺⁺ ou magnésium Mg⁺⁺.

Dans des variantes non illustrées, les cations bivalents 22 sont remplacés par des cations multivalents autres que bivalents, par exemple des cations trivalents tels que des cations d'aluminium Al⁺⁺⁺ ou de chrome Cr⁺⁺⁺.

Dans d'autres variantes non illustrées, la substance à base liquide contenant de la matière biologique est différente de la semence animale diluée, par exemple un milieu de conservation contenant des embryons.

De nombreuses autres variantes sont possibles en fonction de circonstances, et l'on rappelle à cet égard que l'invention ne se limite pas aux exemples décrits et représentés.

## Revendications

1. Paillette pour la conservation d'une dose prédéterminée de substance à base liquide contenant de la matière biologique, comportant un tube (11) s'étendant entre une première extrémité (16) et une seconde extrémité (17) et comportant un bouchon (12) perméable aux gaz et étanche aux liquides, lequel bouchon (12) est disposé dans le tube (11) au voisinage de sa première extrémité (16) et s'étend entre une première extrémité (19) tournée vers la première extrémité (16) du tube (11) et une seconde extrémité (18) tournée vers la seconde extrémité (17) du tube (11) ; **caractérisée en ce qu'**à l'état vide initial de la paillette (10) ledit bouchon (12) comporte un élément (14) imprégné par des cations multivalents (22) au moins au voisinage d'une première extrémité (18 ; 33) tournée vers la seconde extrémité (17) du tube (11).

2. Paillette selon la revendication 1, **caractérisée en ce que** lesdits cations multivalents sont des cations bivalents.

3. Paillette selon la revendication 2, **caractérisée en ce que** lesdits cations bivalents comportent des cations (22) de baryum Ba⁺⁺.

4. Paillette selon la revendication 2, **caractérisée en ce que** lesdits cations bivalents comportent des cations de calcium Ca⁺⁺.

5. Paillette selon l'une quelconque des revendications 1 à 4, **caractérisée en ce que** ledit bouchon (12) comporte deux tampons (13, 14) en substance fibreuse enserrant un agent de scellement (20) formé par une poudre (15) se transformant au contact d'un liquide en une pâte ou gel imperméable (15') adhérent à la paroi du tube pour que le bouchon soit étanche aux liquides.

6. Paillette selon la revendication 5, **caractérisée en ce que**, dans le bouchon (12) de la paillette (10), le tampon (14) situé du côté de la seconde extrémité (17) du tube (11) forme ledit élément imprégné, avec ladite première extrémité (18) de l'élément imprégné qui forme ladite seconde extrémité du bouchon.

7. Paillette selon l'une quelconque des revendications 1 à 5, **caractérisée en ce que** ledit bouchon (12) comporte un tampon barrière (30) perméable aux gaz et aux liquides s'étendant entre une première extrémité (31) tournée vers la première extrémité (17) du tube (11) et une seconde extrémité (32) tournée vers la seconde extrémité (16) du tube (11), avec la première extrémité (33) de l'élément imprégné (14) et la seconde extrémité (32) du tampon barrière (30) qui sont disposées l'une contre l'autre.

8. Paillette selon la revendication 7, **caractérisée en ce que** le tampon barrière (30) est hydrophobe.

9. Paillette selon l'une quelconque des revendications 1 à 8, **caractérisée en ce que** ledit élément imprégné est un tampon (14) entièrement imprégné de cations bivalents (22).

10. Système pour la conservation d'une matière biologique, **caractérisé en ce qu'**il comporte :
- une paillette (10) selon l'une quelconque des revendications 1 à 9 pour la conservation d'une dose prédéterminée de substance à base liquide contenant de ladite matière biologique ; et
- un milieu liquide de dilution (27) pour donner par mélange, dans des conditions prédéterminées, ladite substance à base liquide contenant de la matière biologique (21) ; lequel milieu contient un alginate (23) en solution.

11. Système selon la revendication 10, **caractérisé en ce que** ladite substance à base liquide est de la semence animale diluée et ledit alginate est dans une concentration comprise entre 0,1 et 6 g/l.

12. Système selon la revendication 11, **caractérisée en ce que** ledit alginate est dans une concentration comprise entre 2,5 et 5 g/l.

## Patentansprüche

1. Röhrchen zur Aufbewahrung einer vorbestimmten Dosis einer Substanz auf Flüssigkeitsbasis, die biologisches Material enthält, der ein Rohrstück (11) umfasst, das sich zwischen einem ersten Ende (16) und einem zweiten Ende (17) erstreckt und einen Stopfen (12) umfasst, der gasdurchlässig und flüssigkeitsdicht ist, wobei der Stopfen (12) in dem Rohrstück (11) in der Nähe seines ersten Endes (16) angeordnet ist und sich zwischen einem ersten Ende (19), das dem ersten Ende (16) des Rohrstücks (11) zugewandt ist, und einem zweiten Ende (18), das dem zweiten Ende (17) des Rohrstücks (11) zugewandt ist, erstreckt; **dadurch gekennzeichnet, dass** der Stopfen (12) im anfänglich leeren Zustand des Röhrchens (10) ein Element (14) umfasst, das zumindest in der Nähe eines ersten Endes (18; 33), das dem zweiten Ende (17) des Rohrstücks (11) zugewandt ist, mit mehrwertigen Kationen (22) imprägniert ist.

2. Röhrchen nach Anspruch 1, **dadurch gekennzeichnet, dass** die mehrwertigen Kationen zweiwertige Kationen sind.

3. Röhrchen nach Anspruch 2, **dadurch gekennzeichnet, dass** die zweiwertigen Kationen Bariumkationen Ba⁺⁺ (22) umfassen.

4. Röhrchen nach Anspruch 2, **dadurch gekennzeichnet, dass** die zweiwertigen Kationen Calciumkationen Ca⁺⁺ umfassen.

5. Röhrchen (1) nach einem der Ansprüche 1 bis 4, **dadurch gekennzeichnet, dass** der Stopfen (12) zwei Pfropfen (13, 14) aus faserartigem Material umfasst, die ein Dichtungsmittel (20) einschließen, das aus einem Pulver (15) besteht, das sich bei Kontakt mit einer Flüssigkeit in eine undurchlässige Paste oder ein undurchlässiges Gel (15') verwandelt, das an der Wand des Rohrstücks haftet, so dass der Stopfen flüssigkeitsdicht ist.

6. Röhrchen nach Anspruch 5, **dadurch gekennzeichnet, dass** in dem Stopfen (12) des Röhrchens (10) der Pfropfen (14), der auf der Seite des zweiten Endes (17) des Rohrstücks (11) gelegen ist, das imprägnierte Element bildet, wobei das erste Ende (18) des imprägnierten Elements das zweite Ende des Stopfens bildet.

7. Röhrchen (1) nach einem der Ansprüche 1 bis 5, **dadurch gekennzeichnet, dass** der Stopfen (12) einen gas- und flüssigkeitsdurchlässigen Sperrpfropfen (30) umfasst, der sich zwischen einem ersten Ende (31), das dem ersten Ende (17) des Rohrstücks (11) zugewandt ist, und einem zweiten Ende (32), das dem zweiten Ende (16) des Rohrstücks (11) zugewandt ist, erstreckt, wobei das erste Ende (33) des imprägnierten Elements (14) und das zweite Ende (32) des Sperrpfropfens (30) gegeneinander angeordnet sind.

8. Röhrchen nach Anspruch 7, **dadurch gekennzeichnet, dass** der Sperrpfropfen (30) hydrophob ist.

9. Röhrchen nach einem der Ansprüche 1 bis 8, **dadurch gekennzeichnet, dass** das imprägnierte Element ein Pfropfen (14) ist, der vollständig mit bivalenten Kationen (22) imprägniert ist.

10. System zur Aufbewahrung von biologischem Material, **dadurch gekennzeichnet, dass** es umfasst:
- einen Röhrchen (10) nach einem der Ansprüche 1 bis 9 zur Aufbewahrung einer vorbestimmten Dosis einer Substanz auf Flüssigkeitsbasis, die das biologische Material enthält; und
- ein flüssiges Verdünnungsmedium (27), um durch Mischen unter vorbestimmten Bedingungen die Substanz auf Flüssigkeitsbasis, die biologisches Material (21) enthält, zu ergeben; wobei das Medium ein Alginat (23) in Lösung enthält.

11. Röhrchen nach Anspruch 10, **dadurch gekennzeichnet, dass** die Substanz auf Flüssigkeitsbasis verdünntes Tiersperma ist und das Alginat in einer Konzentration zwischen 0,1 und 6 g/l vorliegt.

12. Röhrchen nach Anspruch 11, **dadurch gekennzeichnet, dass** das Alginat in einer Konzentration zwischen 2,5 und 5 g/l vorliegt.

## Claims

1. A straw for the preservation of a predetermined dose of liquid-based substance containing biological material, comprising a tube (11) extending between a first end (16) and a second end (17) and comprising a liquid-tight, gas-permeable stopper (12), which stopper (12) is disposed in the tube (11) close to its first end (16) and extends between a first end (19) facing towards the first end (16) of the tube (11) and a second end (18) facing towards the second end (17) of the tube (11); **characterized in that** in the initial empty state of said straw said stopper (12) comprises a member (14) impregnated with multivalent cations (22) at least in the neighborhood of a first end (18; 33) facing towards the second end (17) of the tube (11).

2. A straw according to claim 1, **characterized in that** said multivalent cations are divalent cations.

3. A straw according to claim 2, **characterized in that** said divalent cations comprise barium Ba⁺⁺ cations (22).

4. A straw according to claim 2, **characterized in that** said divalent cations comprise calcium Ca⁺⁺ cations.

5. A straw according to any one of claims 1 to 4, **characterized in that** said stopper (12) comprises two plugs (13, 14) made from a fibrous substance enclosing a sealing agent (20) formed by a powder (15) which, on contact with a liquid, transforms into an impermeable paste or gel (15') adhering to the wall of the tube so that the stopper is liquid-tight.

6. A straw according to claim 5, **characterized in that** , in the stopper (12) of the straw (10), the plug (14) situated towards the second end (17) of the tube (11) forms said impregnated member, said first end (18) of the impregnated member forming said second end of the stopper.

7. A straw according to any one of claims 1 to 5, **characterized in that** said stopper (12) comprises a gas-permeable, liquid permeable barrier plug (30) extending between a first end (31) facing towards the first end (17) of the tube (11) and a second end (32) facing towards the second end (16) of the tube (11), the first end (33) of the impregnated member (14) and the second end (32) of the barrier plug (30) being disposed against each other.

8. A straw according to claim 7, **characterized in that** the barrier plug (30) is hydrophobic.

9. A straw according to any one of claims 1 to 8, **characterized in that** said impregnated member is a plug (14) entirely impregnated with divalent cations (22).

10. A system for the preservation of biological material, **characterized in that** it comprises
- a straw (10) according to any one of claims 1 to 9 for the preservation of a predetermined dose of liquid-based substance containing biological material; and
- a liquid dilution extender (27) to give by mixing, in predetermined conditions, said liquid-based substance containing biological material (21); which extender contains an alginate (23) in solution.

11. A system according to claim 10, **characterized in that** said liquid-based substance is diluted animal semen and said alginate is in a concentration comprised between 0.1 and 6 g/l.

12. A system according to claim 11, **characterized in that** said alginate is in a concentration comprised between 2.5 and 5 g/l.
